# EUROPEAN PATENT APPLICATION

(11) **EP 4 582 560 A1**
(43) Date of publication of application: **09.07.2025**
(21) Application number: 22956984.3
(22) Date of filing: 02.09.2022
(51) Int. Cl.: C12Q 1/6886, C12N 15/11, C12Q 1/6858

(54) **DIFFERENTIALLY METHYLATED REGION OF ADHFE1 GENE, KIT, AND USE THEREOF**

(71) Applicant: BGI Genomics Co., Limited, Guangdong 518083 (CN)
(72) Inventor: LI, Zhilong, Shenzhen, Guangdong 518083 (CN); WANG, Yuying, Shenzhen, Guangdong 518083 (CN); PENG, Jiaxi, Shenzhen, Guangdong 518083 (CN); JIANG, Ruijingfang, Shenzhen, Guangdong 518083 (CN); SUN, Jianlong, Shenzhen, Guangdong 518083 (CN)
(74) Representative: Inspicos P/S
(86) International application number: PCT/CN2022/116741
(87) International publication number: WO 2024/045162

(57) **Abstract**

The present invention belongs to the field of bio-pharmaceuticals. Disclosed are a differentially methylated region of an ADHFE1 gene, a detection primer, a probe, a kit, and use of the differentially methylated region in the detection of colorectal cancer or precancerous lesions of colorectal cancer and in the evaluation of prognostic risks of colorectal cancer patients. The differentially methylated region is chr8:67344198-67345563. The differentially methylated region of the present invention can be used for effectively detecting colorectal cancer or precancerous lesions of colorectal cancer, or for evaluating prognostic risks of colorectal cancer patients.

## Description

### Technical Field

The present invention belongs to the field of biomedicine, and relates to differentially methylated region of ADHFE1 gene, kit and use. Specifically, the kit is a kit for detecting the differentially methylated region. Specifically, the use is for detecting colorectal cancer, or for the prognostic risk assessment of patients with colorectal cancer.

### Background Art

Colorectal cancer occurring in the colon and rectum of the lower digestive tract is a common malignant tumor. According to the latest statistics, the number of new cases of colorectal cancer ranks third among men and second among women worldwide, and the number of deaths ranks fourth among men and third among women (Bray et al., 2018). In China, the prevention and control situation of colorectal cancer is also very severe, which ranks third among new cases of malignant tumors (388,000) and fifth among cases of deaths (187,000) (Zheng Rongshou et al., 2019). Colorectal cancer develops slowly, generally going through polyps, adenomas, and intestinal cancer, and it can take up to 5 to 10 years for adenoma to develop into intestinal cancer. If intervention is carried out in the early stages of colorectal cancer, the mortality rate can be significantly reduced. The five-year survival rate of patients with stage I colorectal cancer can reach more than 90%, while the five-year survival rate of patients with stage IV colorectal cancer is less than 20% (Marzieh Araghi et al., 2020).

Traditional colorectal cancer early screening technologies mainly include colonoscopy, fecal occult blood test, tumor markers CEA and CA19-9, etc. These technical means currently have certain limitations. Colonoscopy is the gold standard for colorectal cancer diagnosis. Lens and light source can be inserted from anus, move along cavity to pass through rectum, sigmoid colon and other parts to reach the end of ileum, and images can be transmitted to display in real time for the operating doctor to observe. The images of colonoscope are intuitive and clear, and various lesions such as cancer, polyps, ulcers, and bleeding can be observed. Polyps can also be removed under microscope. However, it is an invasive detection technology with a complicated preparation process, requiring diet control and intestinal cleaning, and the compliance of examinees is low. In addition, it also requires equipment and personnel, and needs to be operated by hospital professionals and anesthesiologists. Some examinees will feel uncomfortable and have the risk of complications (3 to 5 cases/1000 people). Fecal occult blood test is a non-invasive method that detects blood components (hemoglobin) in stool to determine whether there is gastrointestinal bleeding, so that doctors can judge the risk of colorectal cancer. This method is quick and simple, and the compliance of examinees is high. However, this detection method is easily affected by liver, blood products, green leafy vegetables, etc. in the diet, resulting in false positives. At the same time, it has low sensitivity for early colorectal cancer patients with less bleeding, and is prone to missed diagnosis. CEA and other broad-spectrum tumor markers have low specificity, are prone to more false positives, and have limited sensitivity. Therefore, it is in need to establish an accurate, simple and economical early screening method for colorectal cancer.

DNA methylation is an important gene expression regulation mechanism that can regulate gene expression and silencing, and has a significant impact on the occurrence and development of tumors. Abnormal methylation of cancer-related genes often occurs in the early stages of cancer, so DNA methylation signals are considered to be potential early screening markers for tumors. Human fecal samples are mixed with intestinal cell DNA, which carries information such as methylation. By detecting and analyzing this information, the possibility of examinee suffering from colorectal cancer can be inferred. Some genes have been proven to be methylation markers for colorectal cancer detection in feces, such as SFRP2, SEPT9, NDRG4 and SDC2 (Hannes M Müller et al., 2004; Jie Chen et al., 2019), but their performance cannot fully meet clinical needs. For example, the sensitivity and specificity of SFRP2 for colorectal cancer are only 77% (Hannes M Müller et al., 2004).

ADHFE1 encodes a transhydrogenase. The abnormal regulation caused by hypermethylation of this gene can shorten the cycle of colorectal cancer cells and promote cancer cell proliferation (Tae CH, Ryu KJ, Kim SH, Kim HC, Chun HK, Min BH, et al. Alcohol dehydrogenase, iron containing, 1 promoter hypermethylation associated with colorectal cancer differentiation. BMC Cancer. 2013;13:142.).

At present, there is still a need to develop new markers for colorectal cancer detection.

### Contents of the present invention

After in-depth research and creative work, the inventors discovered the differentially methylated region (DMR) of the ADHFE1 gene. The inventors surprisingly found that the differentially methylated region can be used to effectively detect a colorectal cancer or colorectal cancer precancerous lesions, or to assess the prognostic risk of colorectal cancer patients, with good sensitivity and specificity. The present invention is provided as follows:
One aspect of the present invention relates to a differentially methylated region, which is chr8:67344198-67345563 (also referred to as the DMR of the present invention).

The differentially methylated region is a differentially methylated region of ADHFE1 gene.

In some embodiments of the present invention, the differentially methylated region has a base sequence as set forth in SEQ ID NO: 1 or a complementary sequence thereof.

The base sequence of DMR of the present invention:

AGTCTAATCATGGCAAGTTTTAACTATTGATGTAAGAGTTTTAACTATTGATTTT
GCTTTCATCAAAAGCAAAGCCATTCACACAGTATTTAACAAAAACCCTCGGGCTCTG
CTAGGTTCTTGTTTACTGTTAAAACAGTTTAAAGGCAGGGTCTTGATCACTTAAAAT
ACTCTTAGAAGGGAAAGTAACATTTTAAAGGATTAACAGTGGAAAAAGAATATCCC
TGTTCCTCTTCCTTCCCCCAAGTCCTCAACATGTGAAAAATAGAGTTGATAAGTAAG
GAGATTTAAGGCAGAACCCGAGGCCTACGGAGCAGTTACCTTCTACGGCAATTTCA
AGGGTGGATGGTGCGAGCGCCGCTGGGGCAGCTGGCGTTCTGGTTCTTACTCCGTGG
GAAAATGGCCCTGAGCCCGACTGGCTTGAGGCTTAGACAGGTGACCCCGCGAAGCG
GGTGGGCAGGCGCGGCCGAGGGGCGGGAGGCGGGCAGCCTCCGTGATTGGCCGGC
GGAGGGGGCGGTGTAGCGACCCGAGGAGGGAAGAGGACTCCAAGCGCCATGGCCG
CTGCCGCCCGAGCCCGGGTCGCGTACTTGCTGAGGCAACTGCAACGCGCAGCGTGA
GTGCGGGGCCGGCGGGCGGGCAGGGGACCGCAGGGTGCCCACGCAGCCCCCTGGGT
GTGACCCGCTCAGATCCTGCGCAGTCTTCTCCGCTTACTTTCAAGAATTCGGAGAAT
TTGGATACCGCCCTGGGCTCCCACCGCGCGCCAGGCGGAACAGCGAGGTCACAGAG
TGCGCGATGCAGCCTCTCAGGGCGCAGTGAGGCAGCCTTTACTCTGCTTGTCTACCG
TTAAAGAAACTGAGGCCCAGAGAGGACCTGGTCCCACATCGTAGCAGGCAGTGGAG
AGCTTGGCACGGTGGCCAAGACAAATTCATGTATGCAATTGACACATATTTATTGGG
CATATATTATGTGCCAAAAACTATTCTAGGTTCTGGGGATACAAAGATGAAAGGCTC
TGGAGCAGTTTGAGGTCTAGTGGGGAATGGGATGAGGGGACGAGGCTAAAACCGGC
TATAGCAGACTGGGTGTAGAAGGGAAGGATGGATGTGAGAAGTCGCAGAGTCACAC
TTCCTGGCATGTACAGGGAGTCAGGGTATTCAATGTCATGACACGCCTGCCTCCCAG
CAGAAATGTGAGGTACATCCTGGTCACCTGATAAATACTTGCTGACTGTGCAAGTGC
ACAAGTCTGCCTGGGAAACATGGAAAAGTTTGAGCTCACAGGTGCAGATCAGTAGG
AGGAAAGGCGAGGGACTGGCGAGGAACGTCAGGCAGAGAGAAGAGCAACACCAGC
GATGGAGATGCAGTCC (SEQ ID NO: 1)

In some embodiments of the present invention, the differentially methylated region is used to detect a colorectal cancer or a colorectal cancer precancerous lesion, or to assess the prognostic risk of a colorectal cancer patient.

In some embodiments of the present invention, the differentially methylated region has undergone the following treatment:
bisulfite treatment, methylation sensitive endonuclease treatment, methylation restriction endonuclease treatment, or methylation modification enzyme treatment.

The differentially methylated region of the present invention is an ADHFE1 gene methylation marker for colorectal cancer detection.

Another aspect of the present invention relates to a kit, comprising a reagent for detecting the differentially methylated region as described in any one of items of the present invention or a reagent for detecting a methylation site within the differentially methylated region.

In some embodiments of the present invention, in the kit, the reagent comprises:
(1) a primer and a probe for detecting the differentially methylated region or a methylation site within the differentially methylated region;
preferably, the reagent also comprises:
(2) a primer and a probe for an internal reference gene.

In some embodiments of the present invention, in the kit, in (1), the sequence of the primer is selected from SEQ ID NOs: 2 to 81, and the sequence of the probe is selected from SEQ ID NOs: 82 to 102;
preferably, in (1): the sequence of the primer is selected from SEQ ID NOs: 30 to 31, and the sequence of the probe is selected from SEQ ID NO: 90; the sequence of the primer is selected from SEQ ID NOs: 78 to 79, and the sequence of the probe is selected from SEQ ID NO: 102; the sequence of the primer is selected from SEQ ID NOs: 80 to 81, and the sequence of the probe is selected from SEQ ID NO: 96; the sequence of the primer is selected from SEQ ID NOs: 66 to 67, and the sequence of the probe is selected from SEQ ID NO: 97; the sequence of the primer is selected from SEQ ID NOs: 76 to 77, and the sequence of the probe is selected from SEQ ID NO: 99; the sequence of the primer is selected from SEQ ID NOs: 30 to 31, and the sequence of the probe is selected from SEQ ID NO: 86; or the sequence of the primer is selected from SEQ ID NOs: 30 to 31, and the sequence of the probe is selected from SEQ ID NO: NO: 84;
preferably, in (2), the sequence of the primer of the internal reference gene is set forth in SEQ ID NOs: 103 to 104, and the sequence of the probe of the internal reference gene is set forth in SEQ ID NO: 105.

In some embodiments of the present invention, the kit further comprises one or more selected from the following:
a stool sample sampler, a nucleic acid extraction reagent, a methylation detection sample pretreatment reagent and a PCR reagent.

In some embodiments of the present invention, in the kit, the methylation detection sample pretreatment reagent is a bisulfite, a methylation sensitive endonuclease, a methylation restriction endonuclease or a methylation modification enzyme.

Another aspect of the present invention relates to the use of the differential methylation region of the present invention, or the reagent for detecting the differential methylation region or the methylation site within the differential methylation region of in any one of items of the present invention in the manufacture of a medicament for detecting a colorectal cancer or a colorectal cancer precancerous lesion, or for assessing the prognosis risk of a colorectal cancer patient.

In some embodiments of the present invention, in the use, the reagent comprises a primer and a probe for detecting the differentially methylated region or the methylation site in the differentially methylated region as described in any one of items of the present invention.

In some embodiments of the present invention, in the use, the primer and the probe are the primer and the probe as described in the above item (1).

In some embodiments of the present invention, in the use, the reagent further comprises a primer and a probe of an internal reference gene; preferably, the sequence of the primer of the internal reference gene is set forth in SEQ ID NOs: 103 to 104, and the sequence of the probe of the internal reference gene is set forth in SEQ ID NO: 105.

Another aspect of the present invention relates to a method for detecting a colorectal cancer or a colorectal cancer precancerous lesion or a method for assessing the prognosis risk of a colorectal cancer patient, comprising the following steps:
detecting the content of the differentially methylated region as described in any one of items of the present invention or the content of a fragment thereof; wherein the fragment comprises one or more methylation sites, for example, 1 to 20, 1 to 15, 1 to 10, 1 to 5, 1, 2, 3, 4 or 5 methylation sites, in the differentially methylated region.

Those skilled in the art can understand that the detected content of the fragment in the differentially methylated region, for example, the content of the fragment as detected by PCR method, can represent the content of the differentially methylated region.

In some embodiments of the present invention, in the method, the content of the methylated molecule in the differentially methylated region as described in any one of items of the present invention is detected by a fluorescence quantitative PCR method.

In some embodiments of the present invention, in the method,
the content of human DNA in a sample is evaluated by an internal reference gene Ct value; when the internal reference gene Ct value is >37, the sample is judged to have too low human DNA content and the detection fails; when the internal reference gene Ct value is ≤37, the sample is evaluated to be qualified and can be analyzed later.

In some embodiments of the present invention, in the method,
if △Ct ≤ a cutoff value (for example, the cutoff value is 10), it is determined to be a high risk of colorectal cancer, otherwise it is determined to be a low risk;
wherein △Ct = (Ct value of the differentially methylated region or Ct value of the amplified fragment) - Ct value of the reference gene;
wherein, the amplified fragment comprises one or more methylation sites, for example, 1 to 20, 1 to 15, 1 to 10, 1 to 5, 1, 2, 3, 4 or 5 methylation sites, in the differentially methylated region.

In some embodiments of the present invention, in the method, the sample is a human tissue sample, a blood sample, a cell sample, a secretion sample or an excrement sample such as a stool sample.

According to some embodiments of the present invention, a stool sample is used as a detection object. In this way, the stool sample contains intestinal exfoliated cells, meets the requirements of the detection sample, is non-invasive, and is more patient-friendly.

According to some embodiments of the present invention, the optional detection means include but are not limited to: Sanger sequencing, pyrophosphate sequencing, high-throughput sequencing, nucleic acid mass spectrometry, PCR, fluorescence quantitative PCR, ddPCR, etc. Since the nucleic acid sequence has not changed, methylation modification is difficult to be directly detected, the nucleic acid often needs to be pretreated during the experiment. The pretreatment methods include but are not limited to: bisulfite treatment, methylation-sensitive endonuclease treatment, methylation restriction endonuclease treatment or methylation modification enzyme treatment. At the same time, the third-generation sequencing technology has been proven to be useful for directly determining the methylation modification state of DNA, and with the development of technology, it can also be used as a detection method in the future. Fluorescence quantitative PCR technology is one of the commonly used and mature detection technologies for molecular diagnosis. It has the characteristics of rapidity, accuracy and low cost. In the preferred embodiment of the present invention, a method of bisulfite treatment combined with fluorescence quantitative PCR is adopted, and several methylation-specific PCR primers and probes are designed for the ADHFE1 gene, which can specifically identify methylated DNA molecules, realize the detection of the methylation state of the sample, and then evaluate the cancer risk of the subject.

By performing whole genome methylation sequencing and targeted methylation sequencing on colorectal cancer tissue and control tissue samples, the methylation differential regions highly associated with colorectal cancer were obtained, among which the methylation rate difference of the DMR (chr8:67344198,67345563) of the ADHFE1 gene was particularly significant. Primers and probes were designed for the above regions, which can be used to quickly and accurately detect the methylation status of the ADHFE1 gene. The present invention provides several primer and probe sequences, as shown in Table 1:

**Table 1: ADHFE1 gene primers, probes**

| SEQ ID NO: | Sequence (5'-3') | Description |
|---|---|---|
| 2 | TCGTTGGGGTAGTTGGC | ADHFE1 gene primers |
| 3 | CCTATCTAAACCTCAAACCAATCG | |
| 4 | GTTTTGGGTTTTTATCGCGC | ADHFE1 gene primers |
| 5 | GCCCTAAAAAACTACATCGCG | |
| 6 | AGGTTTAGATAGGTGATTTCGC | ADHFE1 gene primers |
| 7 | AAACTACCCGCCTCCC | |
| 8 | TTGGCGTTTTGGTTTTTATTTC | ADHFE1 gene primers |
| 3 | CCTATCTAAACCTCAAACCAATCG | |
| 9 | GATTTAAGGTAGAATTCGAGGTTTAC | ADHFE1 gene primers |
| 10 | CACGAAATAAAAACCAAAACG | |
| 11 | CGTAGTTTTTTGGGTGTGATTC | ADHFE1 gene primers |
| 12 | AAAACGATATCCAAATTCTCCG | |
| 13 | CGTATTTTGTGATTTCGTTGTTTC | ADHFE1 gene primers |
| 14 | AAAAATTCGAAAAATTTAAATACCG | |
| 15 | CGTTTGTTTATTCGTTTCGC | ADHFE1 gene primers |
| 16 | CGCCGCTAAAACAACTAACG | |
| 17 | CGTTTTGAGAGGTTGTATCGC | ADHFE1 gene primers |
| 14 | AAAAATTCGAAAAATTTAAATACCG | |
| 18 | GTTTAGGGCGGTATTTAAATTTTTC | ADHFE1 gene primers |
| 19 | ATATAACCCGCTCAAATCCTACG | |
| 20 | TGTATCGCGTATTTTGTGATTTC | ADHFE1 gene primers |
| 21 | CCGCTTACTTTCAAAAATTCG | |
| 22 | GGTAATTTTAAGGGTGGATGGTG | ADHFE1 gene primers |
| 23 | TCACCTATCTAAACCTCAAACCA | |
| 24 | GGGGTTATTTGTTTAAGTTTTAAGTTAGT | ADHFE1 gene primers |
| 25 | AAACAATTACCTTCTACRACAATTTCA | |
| 26 | AAGTAAGGAGATTTAAGGTAGAATT | ADHFE1 gene primers |
| 27 | TTAAAATCCTCTTCCCTCCTC | |
| 28 | ATATGTGAAAAATAGAGTTGATAAGTAAGG | ADHFE1 gene primers |
| 29 | CTTAAAATCCTCTTCCCTCCTC | |
| 30 | GATGGTGCGAGCGTC | ADHFE1 gene primers |
| 31 | CTATCTAAACCTCAAACCAATCG | |
| 30 | GATGGTGCGAGCGTC | ADHFE1 gene primers |
| 32 | TACACCGCCCCCTCC | |
| 30 | GATGGTGCGAGCGTC | ADHFE1 gene primers |
| 33 | TCCGCCGACCAATCAC | |
| 30 | GATGGTGCGAGCGTC | ADHFE1 gene primers |
| 34 | CCGAACTCGAACGACAAC | |
| 35 | AYGGTAATTTTAAGGGTGGATGGTGYGAGYGT | ADHFE1 gene primers |
| 36 | CRCCCCCTCCRCCRACCAATCACRAAAACT | |
| 37 | AAGTAAGGAGATTTAAGGTAGAATTYGAGGTT TAYGGAGTAGT | ADHFE1 gene primers |
| 38 | ACCATAACRCTTAAAATCCTCTTCCCTCCTCRA ATCRCT | |
| 39 | GGAGATTTAAGGTAGAATTCGAGGTTTAC | ADHFE1 gene primers |
| 40 | CTCGAACGACAACGACCATAAC | |
| 41 | GAATTCGAGGTTTACGGAGTAGT | ADHFE1 gene primers |
| 42 | CTCCTCGAATCGCTACACC | |
| 43 | TTTAAGGGTGGATGGTGCGAGC | ADHFE1 gene primers |
| 44 | CTCAACAAATACGCGACCC | |
| 45 | CGGTTAATTACGGAGGTTGTTC | ADHFE1 gene primers |
| 46 | CCTACGAAACAATTACCTTCTACGA | |
| 45 | CGGTTAATTACGGAGGTTGTTC | ADHFE1 gene primers |
| 47 | AAAACAAAACCCGAAACCTAC | |
| 48 | TAGTAAGTACGCGATTCGGGTTC | ADHFE1 gene primers |
| 49 | GAAACCTACGAAACAATTACCTTCTAC | |
| 50 | CGGAGTAGTTATTTTTTACGG | ADHFE1 gene primers |
| 51 | AACGACAACGACCATAAC | |
| 50 | CGGAGTAGTTATTTTTTACGG | ADHFE1 gene primers |
| 52 | GTTACAATTACCTCAACAAATACG | |
| 53 | GGTAGAATTCGAGGTTTACG | ADHFE1 gene primers |
| 54 | TCTTCCCTCCTCGAATCG | |
| 53 | GGTAGAATTCGAGGTTTACG | ADHFE1 gene primers |
| 55 | CAATCACGAAAACTACCCG | |
| 30 | GATGGTGCGAGCGTC | ADHFE1 gene primers |
| 56 | GAACTCGAACGACAACG | |
| 30 | GATGGTGCGAGCGTC | ADHFE1 gene primers |
| 57 | AACTCGAACGACAACGAC | |
| 30 | GATGGTGCGAGCGTC | ADHFE1 gene primers |
| 58 | CGTTACAATTACCTCAACAAATACG | |
| 30 | GATGGTGCGAGCGTC | ADHFE1 gene primers |
| 59 | TTAAAATCCTCTTCCCTCCTCG | |
| 30 | GATGGTGCGAGCGTC | ADHFE1 gene primers |
| 60 | ATCCTCTTCCCTCCTCG | |
| 61 | AGGTAGAATTCGAGGTTTACG | ADHFE1 gene primers |
| 62 | TACCCACCCGCTTCG | |
| 63 | TGGGAAAATGGTTTTGAGTTCG | ADHFE1 gene primers |
| 59 | TTAAAATCCTCTTCCCTCCTCG | |
| 64 | GAGGGAAGAGGATTTTAAGCG | ADHFE1 gene primers |
| 65 | CCAAAACGATATCCAAATTCTCCG | |
| 66 | GGATCGTAGGGTGTTTACG | ADHFE1 gene primers |
| 67 | CTACGCCCTAAAAAACTACATCG | |
| 68 | GAATTCGGAGAATTTGGATATCG | ADHFE1 gene primers |
| 69 | AACTCTCCACTACCTACTACG | |
| 70 | GCGTATTTGTTGAGGTAATTGTAACG | ADHFE1 gene primers |
| 71 | ACTCTATAACCTCGCTATTCCG | |
| 72 | AGGAGGGAAGAGGATTTTAAGCG | ADHFE1 gene primers |
| 73 | CCCAAAACGATATCCAAATTCTCCG | |
| 74 | GGGGATCGTAGGGTGTTTACG | ADHFE1 gene primers |
| 75 | CTCACTACGCCCTAAAAAACTACATCG | |
| 76 | TCGGTTAATTACGGAGGTTGTTCG | ADHFE1 gene primers |
| 77 | AACCTACGAAACAATTACCTTCTACG | |
| 78 | GAGTTTAGGGCGGTATTTAAATTTTTCG | ADHFE1 gene primers |
| 79 | AAACTCCAAACGCCATAACCG | |
| 80 | TTAAGCGTTATGGTCGTTGTCG | ADHFE1 gene primers |
| 81 | GCGCGATAAAAACCCAAAACG | |
| 82 | TCAAAACCATTTTCCCACGAAATAAA | ADHFE1 gene probe |
| 83 | CTCTATAACCTCGCTATTCCGCCT | ADHFE1 gene probe |
| 84 | CCCACGAAATAAAAACCAAAACGCCAAC | ADHFE1 gene probe |
| 85 | ACTCAAAACCATTTTCCCACGAA | ADHFE1 gene probe |
| 86 | ACTCAAAACCATTTTCCCACGAAAT | ADHFE1 gene probe |
| 87 | AACGAAAAACGCCACTACCCGATACGA | ADHFE1 gene probe |
| 88 | AACGCCACTACCCGATACGAACTACACC | ADHFE1 gene probe |
| 89 | AAAAACCAAAACGCCAACTACCCCA | ADHFE1 gene probe |
| 90 | CAAAACGCCAACTACCCCAACGAC | ADHFE1 gene probe |
| 91 | TGCGAGCGTCGTTGGGGTA | ADHFE1 gene probe |
| 92 | TGGTCGTTGTCGTTCGAGTTCGG | ADHFE1 gene probe |
| 93 | CCTCGCTATTCCGCCTAACGCG | ADHFE1 gene probe |
| 94 | ACTACGCAAAATCTAAACGAATCA | ADHFE1 gene probe |
| 95 | TATGGTCGTTGTCGTTCGAGTT | ADHFE1 gene probe |
| 96 | TACGTAAACACCCTACGATCCC | ADHFE1 gene probe |
| 97 | TTCGTTTAGATTTTGCGTAG | ADHFE1 gene probe |
| 98 | AACGATATCCAAATTCTCCGAAT | ADHFE1 gene probe |
| 99 | TTCGTTTTTCGGTCGCGTTTGTT | ADHFE1 gene probe |
| 100 | ATACGAACGCCGCTAAAACAACTAACGTTCT | ADHFE1 gene probe |
| 101 | TACCGCCCGAACCCGAATCGCGTA | ADHFE1 gene probe |
| 102 | AAGCGGAGAAGATTGCGTA | ADHFE1 gene probe |

After pretreatment, human biological samples can be used to specifically amplify methylated nucleic acid fragments using optimized primers, probes and PCR reaction systems. Obvious amplification bands appear in highly methylated samples, while weak or no amplification occurs in lowly methylated samples. The methylation status of the sample can be determined by the Ct value and curve. For the detection of ADHFE1 methylation status, reference gene primers and probes including but not limited to those described in Table 2 can also be added to detect human DNA in the sample to obtain the corresponding Ct values, and the methylation status of the sample can be determined by calculating the difference (△Ct) between the ADHFE1 gene DMR Ct value and the reference gene Ct value. Furthermore, by detecting samples from several cancer patients and non-cancer patients, a cutoff value (also called a positive judgment value, which can be obtained by drawing an ROC curve, for example) for determining the risk of cancer can be obtained. By comparing the detection results with the cutoff value, the risk of suffering cancer in the subject can be evaluated.

**Table 2: Sequence table of internal reference gene primers and probes**

| SEQ ID NO: | Sequence (5'-3') | Description |
|---|---|---|
| 103 | TTTAGGAGTGAGTGGAAGATAGA | Internal reference gene primers |
| 104 | AAACCACACCATCCTAATTACCT | |
| 105 | CCCAAAACACATTTCTTCCATTC | Internal reference gene probe |

The optimized primers, probes and PCR reaction system can form an ADHFE1 gene methylation detection kit, and the kit can be further used for cancer risk assessment.

Another aspect of the present invention relates to the use of the differentially methylated region of the present invention in preparing a product for detecting a colorectal cancer or a colorectal precancerous lesion.

In the present invention, if not otherwise specified, the term "colorectal cancer" refers to colon cancer and/or rectal cancer, also briefly referred to as "intestinal cancer".

In the present invention, if not otherwise specified, the term "early colorectal cancer" refers to stage I or stage II colorectal cancer.

In the present invention, if not otherwise specified, detecting a differentially methylated region or detecting a fragment thereof refers to detecting the content of a differentially methylated region or detecting the content of a fragment thereof; wherein the fragment comprises one or more methylation sites within the differentially methylated region.

In the present invention, if not otherwise specified, detecting a methylation site within a differentially methylated region refers to detecting the content of a methylation site within a differentially methylated region or detecting the methylation rate of a methylation site within a differentially methylated region.

### Beneficial Effects of the Invention

The present invention achieves one or more of the following technical effects:
(1) capable of realizing rapid detection of the methylation status of ADHFE1 gene;
(2) capable of detecting, diagnosing or screening colorectal cancer, or being applied to the prognostic risk assessment of colorectal cancer, with high sensitivity and/or specificity; preferably, with both high sensitivity and specificity;
(3) capable of being applied to the detection, diagnosis or screening of early colorectal cancer or colorectal precancerous lesions, with high sensitivity and/or specificity; preferably, with both high sensitivity and specificity.

### Brief Description of the Drawings

Figure 1 shows the differentially methylated gene regions of colorectal cancer.
Figure 2 shows the performance comparison of colorectal cancer-specific methylation genes in detection of colorectal cancer samples and non-colorectal cancer control samples.

### Specific Models for Carrying Out the present invention

The technical solution of the present invention will be described in detail in conjunction with the examples, but those skilled in the art will understand that the following examples are only used to illustrate the present invention and should not be regarded as limiting the scope of the present invention. If the specific conditions were not specified in the examples, they were carried out according to conventional conditions or conditions recommended by the manufacturer. The reagents or instruments used without indicating the manufacturer were all conventional products that could be purchased commercially.

Some relevant definitions refer to the Colorectal Cancer Diagnosis and Treatment Guidelines 2021, the Chinese Society of Clinical Oncology (CSCO), and some relevant contents are quoted as follows in Table 3:

**Table 3**

| | |
|---|---|
| Colorectal cancer | Colon cancer or rectal cancer confirmed by biopsy pathological diagnosis. |
| Advanced adenoma | Carcinoma in situ and intramucosal carcinoma, with diameter >= 1 cm, or with villous structure, or with high-grade neoplasia, with severe dysplasia. |
| Adenoma patients | Patients with adenoma confirmed by pathological diagnosis. |
| Polyp patients | Patients with inflammatory polyps or hyperplastic polyps confirmed or unconfirmed by pathological diagnosis. |
| Other intestinal disease patients | Including patients with non-polyp lesions such as enteritis, diverticulum, and colon melanosis. |

The standard for staging colorectal cancer adopts AJCC.

### Example 1: Discovery of DMR of colorectal cancer-specific methylated genes

### 1. Extraction of tissue samples:

DNeasy Blood & Tissue Kit (Qiagen, #69506) was used to extract DNA from 38 pairs of colorectal cancer sample and para-cancerous tissue sample from 38 people, wherein each pair of colorectal cancer sample and para-cancerous tissue sample came from the same person. Quantification was performed using the Qubit 3.0 system (Invitrogen, USA).

### 2. DNA fragmentation:

200 ng of the extracted DNA was taken, and added with 1 ng of Unmethylated lambda DNA (PROMEGA, #D1521) for subsequent C-U conversion quality control. The DNA was fragmented using an ultrasonic fragmenter, and then the DNA was fragmented using AMPure XP (AGENCOURT, #A63882) for selection of DNA fragments so that the DNA fragment size was concentrated around 160 bp.

### 3. Library construction:

KAPA HyperPlus Library Preparation Kit (KAPA, #KK8510) was used for library construction, and EZ DNA Metlylation-Gold Kit (Zymo Research, #D5006) was used to perform bisulfite conversion on the adapter ligation product. The specific operation was referred to the kit instructions. The adapters used in the adapter ligation step and the PCR primers used in the PCR step were replaced with adapters and primers suitable for the MGISEQ platform. The adapters needed to be methylated (for example, methylated dCTP was used to synthesize the adapters) to avoid changes in sequence caused by bisulfite treatment.

### 4. High-throughput sequencing:

PE100 sequencing was performed using MGISEQ-2000 (MGI).

### 5. Data analysis

The sequencing data was subjected to quality control, filtration, alignment, and calculation (Non-invasive lung cancer diagnosis and prognosis based on multi-analyte liquid biopsy, Chen Kezhong et al., 2020), and the methylation rate of each CpG site was calculated. By comparing the similarities and differences in the methylation rates of CpG sites in colorectal cancer tissue and para-cancerous tissue, 373 differentially methylated regions (DMRs) between cancer tissue and para-cancerous tissue, i.e., colorectal cancer-specific methylation regions, were identified by the hierarchical Bayes method.

Specific site methylation rate = methylation reads sequencing depth / total sequencing depth.

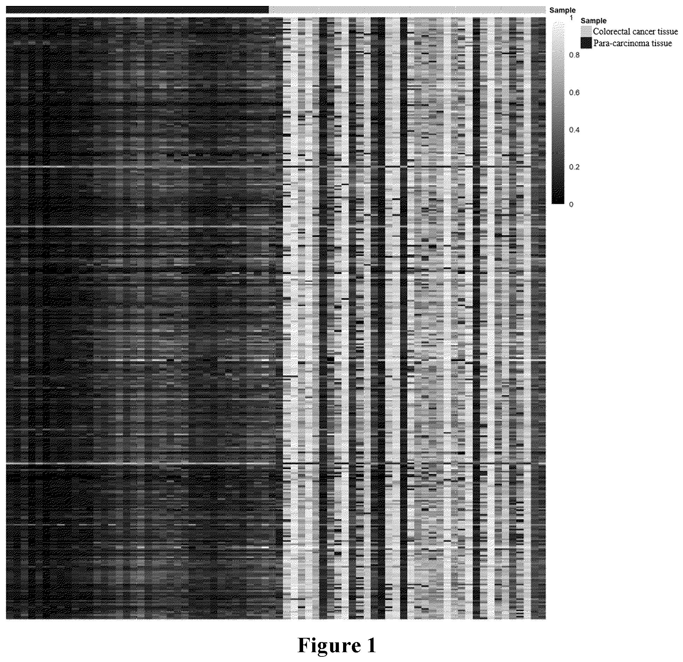

Figure 1 showed a heat map of 373 DMRs found based on 38 pairs of colorectal cancer tissue and para-cancerous tissue, wherein colorectal cancer tissue was on the right and para-cancerous tissue was on the left. The DMRs found included 371 highly methylated DMRs and 2 lowly methylated DMRs. Each grid represented the DMR methylation rate of the corresponding sample at the site, ranging from 0 to 1. The closer the methylation rate was to 0, the darker the color was. The inventors found that the methylation rate difference of the DMR (chr8: 67344198-67345563) of the ADHFE1 gene was particularly significant.

### Example 2: A colorectal cancer detection kit based on ADHFE1 gene DMR

This example aimed to develop a colorectal cancer detection kit based on ADHFE1 gene methylation. In Example 1, the inventors found that the DMR of the ADHFE1 gene (chr8:67344198-67345563) could be used as a marker for colorectal cancer detection. For this DMR, the inventors designed several pairs of methylation-specific primers and probes. After primer screening and reaction condition optimization, stool samples from 137 colorectal cancer patients, 96 healthy people and 23 polyp patients were further detected to determine the performance of the ADHFE1 gene methylation marker for colorectal cancer detection.

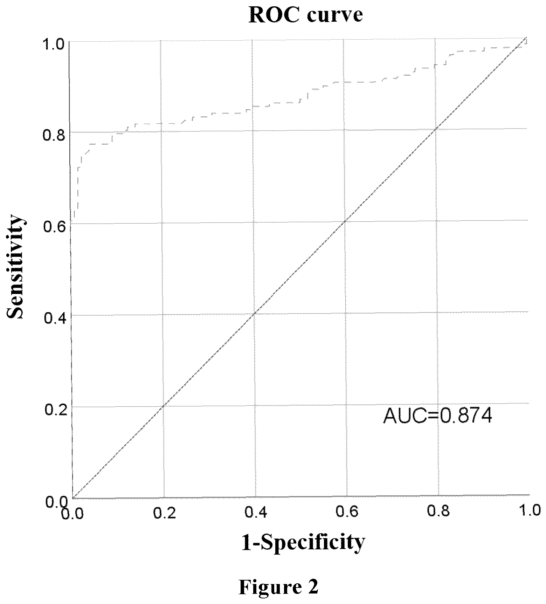

The specific steps were as follows:
1. Sample collection: Disposable stool sample samplers (BGI) were used to collect stool samples from the subjects.
2. Nucleic acid extraction: Stool sample nucleic acid extraction kit (BGI) was used to extract stool sample DNA.
3. Bisulfite conversion: Methylation detection sample pretreatment kit (BGI) was used to perform C-U conversion on DNA.
4. qPCR detection: The reaction system comprised 1x PCR Buffer, 2 to 5 mM magnesium ions, 0.2 to 0.8 mM dNTP, 0.2 µM internal reference gene primers (SEQ ID NO: 103, SEQ ID NO: 104), 0.2 µM ADHFE1 gene primers (SEQ ID NO: 30, SEQ ID NO: 31), 50 nM internal reference gene probe (SEQ ID NO: 105), 50 nM ADHFE1 gene probe (SEQ ID NO: 90) and 1 unit of Taq polymerase. The reaction comprised pre-denaturation at 95°C for 10min, and then 40 cycles of denaturation at 95°C for 30s, annealing at 55°C for 30s and extension at 72°C for 30s. The detection was performed using Hongshi 96S qPCR instrument, with the baseline and threshold set as default, and the fluorescence signal was collected at the end of each cycle.
5. Result analysis: The content of human DNA in the sample was evaluated by using an internal reference gene Ct value; when the Ct value was >37, the human DNA content of the sample was judged to be too low, and the detection failed; when the Ct value was ≤37, subsequent analysis could be performed. The Ct value difference △Ct between the ADHFE1 gene and the internal reference gene was calculated, and the ROC curve was drawn according to the △Ct value (Figure 2). The AUC of the ADHFE1 gene (specifically, the ADHFE1 gene DMR was detected) for colorectal cancer detection reached 0.874. The best positive judgment value was selected according to the Youden Index (YI). The positive judgment value of the kit was set to 10. When △Ct ≤10, it was judged to have a high risk of colorectal cancer, otherwise it was judged to have a low risk. According to the above judgment rules, a total of 106 samples of colorectal cancer patients were detected to have high risk, 89 samples of healthy people were detected to have low risk, and a total of 22 samples of polyps were detected to have low risk. The performance statistics were shown in Table 4.

**Table 4: Detection performance of colorectal cancer detection kit based on ADHFE1 gene DMR**

| Sample Type | Total number of samples | Prediction results | | Sensitivity for colorectal cancer | Specificity | Specificity of total healthy samples and polyp samples |
|---|---|---|---|---|---|---|
| | | High risk | Low risk | | | |
| Colorectal cancer | 137 | 106 | 31 | 77.37% | / | / |
| Healthy people | 96 | 7 | 89 | / | 92.71% | 93.28% |
| Polyps | 23 | 1 | 22 | / | 95.65% | |

According to the above method, the inventors also used some other primers and probe combinations in Table 1 to test the above samples, and all brought good detection results. The specific results were shown in Table 5 below.

**Table 5**

| Primer sequence No. | Probe sequence No. | Colorectal cancer sensitivity | Specificity of combined healthy samples and polyp samples |
|---|---|---|---|
| 78, 79 | 102 | 71.53% | 92.44% |
| 80, 81 | 96 | 75.91% | 88.24% |
| 66, 67 | 97 | 70.80% | 96.64% |
| 76, 77 | 99 | 72.26% | 96.64% |
| 30, 31 | 86 | 72.26% | 89.92% |
| 30, 31 | 84 | 74.45% | 89.92% |

Although the specific embodiments of the present invention have been described in detail, those skilled in the art will understand that various modifications and substitutions can be made to those details based on all the teachings disclosed, and these changes are within the scope of protection of the present invention. The full scope of the present invention is given by the attached claims and any equivalents thereof.

## Claims

1. A differentially methylated region, which is chr8:67344198-67345563.

2. The differentially methylated region according to claim 1, which has a base sequence as set forth in SEQ ID NO: 1 or a complementary sequence thereof.

3. The differentially methylated region according to any one of claims 1 to 2, which is used to detect a colorectal cancer or a colorectal cancer precancerous lesion, or to assess the prognostic risk of a colorectal cancer patient.

4. The differentially methylated region according to any one of claims 1 to 3, which has undergone the following treatment:
a bisulfite treatment, a methylation sensitive endonuclease treatment, a methylation restriction endonuclease treatment or a methylation modification enzyme treatment.

5. A kit, comprising a reagent for detecting the differentially methylated region according to any one of claims 1 to 4 or a reagent for detecting a methylation site within the differentially methylated region.

6. The kit according to claim 5, wherein the reagent comprises:
(1) a primer and a probe for detecting the differentially methylated region or a methylation site within the differentially methylated region;
preferably, the reagent further comprises:
(2) a primer and a probe for an internal reference gene.

7. The kit according to claim 6, wherein:
in (1), the sequence of the primer is selected from SEQ ID NOs: 2 to 81, and the sequence of the probe is selected from SEQ ID NOs: 82 to 102;
preferably, in (1): the sequence of the primer is selected from SEQ ID NOs: 30 to 31, and the sequence of the probe is selected from SEQ ID NO: 90; the sequence of the primer is selected from SEQ ID NOs: 78 to 79, and the sequence of the probe is selected from SEQ ID NO: 102; the sequence of the primer is selected from SEQ ID NOs: 80 to 81, and the sequence of the probe is selected from SEQ ID NO: 96; the sequence of the primer is selected from SEQ ID NOs: 66 to 67, and the sequence of the probe is selected from SEQ ID NO: 97; the sequence of the primer is selected from SEQ ID NOs: 76 to 77, and the sequence of the probe is selected from SEQ ID NO: 99; the sequence of the primer is selected from SEQ ID NOs: 30 to 31, and the sequence of the probe is selected from SEQ ID NO: 86; or the sequence of the primer is selected from SEQ ID NOs: 30 to 31, the sequence of the probe is selected from SEQ ID NO: 84;
preferably, in (2), the sequence of the primer of the internal reference gene is set forth in SEQ ID NOs: 103 to 104, and the sequence of the probe of the internal reference gene is set forth in SEQ ID NO: 105.

8. The kit according to any one of claims 5 to 7, which further comprises one or more selected from the following:
a stool sample sampler, a nucleic acid extraction reagent, a methylation detection sample pretreatment reagent, and a PCR reagent.

9. The kit according to claim 8, wherein the methylation detection sample pretreatment reagent is a bisulfite, a methylation sensitive endonuclease, a methylation restriction endonuclease, or a methylation modifying enzyme.

10. Use of the differentially methylated region according to any one of claims 1 to 4, or a reagent for detecting the differentially methylated region according to any one of claims 1 to 4, or a methylation site in the differentially methylated region in the manufacture of a medicament for detecting a colorectal cancer or a colorectal cancer precancerous lesion, or for assessing the prognosis risk of a colorectal cancer patient.

11. The use according to claim 10, wherein the reagent comprises a primer and a probe for detecting the differentially methylated region according to any one of claims 1 to 4, or a methylation region site within the differentially methylated region.

12. A method for detecting a colorectal cancer or a colorectal cancer precancerous lesion or a method for assessing the prognosis risk of a colorectal cancer patient, comprising the following steps:
detecting the content of the differentially methylated region according to any one of claims 1 to 4 or a fragment thereof; wherein the fragment comprises one or more methylation sites in the differentially methylated region.

13. The method according to claim 12, wherein the content of the differentially methylated region according to any one of claims 1 to 4 is detected by a fluorescent quantitative PCR method.

14. The method according to claim 13, wherein
the content of human DNA in a sample is evaluated by an internal reference gene Ct value, when the internal reference gene Ct value is >37, the sample is judged to have too low human DNA content and the detection fails, and when the internal reference gene Ct value is ≤37, the sample is evaluated to be qualified and can be analyzed later.

15. The method according to any one of claims 13 to 14, wherein
if △Ct ≤ a cutoff value, it is determined to have a high risk of colorectal cancer, otherwise it is determined to have a low risk;
wherein △Ct = (Ct value of the differentially methylated region or Ct value of the amplified fragment) - Ct value of the reference gene;
wherein, the amplified fragment comprises one or more methylation sites in the differentially methylated region.

16. The method according to any one of claims 13 to 15, wherein the sample is a human tissue sample, a blood sample, a cell sample, a secretion sample or an excrement sample such as a fecal sample.

17. Use of the differentially methylated region according to any one of claims 1 to 4 in the manufacture of a product for detecting a colorectal cancer or a colorectal precancerous lesion.
